# EUROPEAN PATENT APPLICATION

(11) **EP 4 559 512 A1**
(43) Date of publication of application: **28.05.2025**
(21) Application number: 23842092.1
(22) Date of filing: 30.06.2023
(51) Int. Cl.: A61N 1/36, A61N 1/375

(54) **TRANSMISSION MODULE AND ELECTRICAL STIMULATION DEVICE**

(30) Priority: 19.07.2022 CN 202210852731
(71) Applicant: Sceneray Co., Ltd, Suzhou, Jiangsu 215123 (CN)
(72) Inventor: YAN, Hao, Suzhou, Jiangsu 215123 (CN)
(74) Representative: Cabinet Beaumont
(86) International application number: PCT/CN2023/104973
(87) International publication number: WO 2024/017027

(57) **Abstract**

Provided are a transmission module (100) and an electrical stimulation device. The transmission module (100) includes a contact assembly (1) including an integral bracket (11)provided with spaced-apart partition-grooves (111) and connecting members (12) each disposed in one of the partition-grooves, first wires (2) each electrically connected to one or more connecting members (12), an external tube (3), and a locking member (4). The integral bracket (11) has an accommodation hole (112), and an extension member (9) is inserted from the rear end of the transmission module (100) into the accommodation hole (112). The external tube (3) is sleeved outside the contact assembly (1). The locking member (4) is sealingly connected to a second front end (34) of the external tube (3). The locking member (4) has a first through hole (41), first wires (2) pass through the first through hole (41) of the locking member (4) and are drawn out from a first front end (101) of the transmission module (100), and the locking member (4) tightly holds the first wires (2). The partition-grooves (111) are provided on the integral bracket (11) and used for fixing the connecting members (12). The assembly dimension of the integral bracket (11) does not increase with the number of modules, the tolerance of modules during assembly can be prevented from increasing, and the locking member (4) can tightly hold the first wires (2) to prevent the first wires from detaching.

## Description

This application claims priority to Chinese Patent Application No. CN202210852731.3 filed on Jul. 19, 2022, which is incorporated herein by reference in its entirety.

### TECHNICAL FIELD

The present application relates to the technical field of connectors and, in particular, to a transmission module and an electrical stimulation device.

### BACKGROUND

Implantable pulse generators are implanted at different positions in the human body to send electrical pulse signals to specific tissues in the human body, thereby treating or improving the symptoms of related diseases, which is a reversible neuromodulation method that can effectively improve the patient's nerve recovery and help the patient recover through nerve stimulation.

A transmission module is required to connect the related pulse generator to the electrode. The transmission module connects the wires that transmit the signals generated by the pulse generator to the extension member of the electrode through the modules inside the transmission module. The related modules are separated, and the isolation and sealing between the modules are achieved through separate flexible waterproof sealing rings. As a result, as the number of modules required for nerve stimulation gradually increases, the dimension after assembly becomes larger and larger, eventually affecting the surgical outcome and user experience. In addition, when the wires are subjected to external forces, the connection between the wires and the modules is likely to fail, resulting in the inability to achieve an electrical connection.

Therefore, a new transmission module is needed to solve the preceding problems.

### SUMMARY

The present application provides a transmission module and an electrical stimulation device to solve the preceding problems.

The object of the present application is achieved by the technical solutions below.

The present application provides a transmission module. The transmission module has a first front end and a first rear end opposite to each other, and the transmission module includes a contact assembly, multiple first wires, an external tube, and a locking member.

The contact assembly includes an integral bracket and multiple connecting members, where the integral bracket is provided with multiple spaced-apart partition-grooves, each of the multiple connecting members is disposed in a respective one of the multiple partition-grooves of the integral bracket, the multiple connecting members are insulated from each other, and the integral bracket has an accommodation hole, and an extension member is inserted from the first rear end of the transmission module into the accommodation hole. Each of the multiple first wires is electrically connected to one or more of the multiple connecting members. The external tube is sleeved outside the contact assembly. The locking member is sealingly connected to the second front end of the external tube, where the locking member has a first through hole, the multiple first wires pass through the first through hole of the locking member and are drawn out from the first front end of the transmission module, and the locking member tightly holds the multiple first wires.

In a possible implementation, the inner side of the second front end of the external tube is threadedly connected to or snap-fit with the locking member.

In a possible implementation, the integral bracket has a conductive member connected in each of the multiple partition-grooves, a groove for fixing a connecting member of the multiple connecting members and a hole for the extension member to pass through are provided on an inner side of the conductive member, the conductive member connects a first wire of the multiple first wires to the connecting member, a first transition surface for arranging the multiple first wires are disposed on an outer surface of the integral bracket, part of a surface of the conductive member is exposed from the first transition surface of the integral bracket and is used for connecting the first wire, and the connecting member is a coil spring.

In a possible implementation, the conductive member includes a wire tube for connecting the first wire, the wire tube is disposed on a second transition surface of the conductive member, and the edge of the second transition surface of the conductive member smoothly transitions to the outer surface of the integral bracket so that the inner circumferential surface of the external tube applies a force to the edge of the second transition surface of the conductive member and presses the conductive member into the integral bracket.

In a possible implementation, the external tube includes a sealing sleeve, the sealing sleeve includes an annular portion and a tube portion, the annular portion and the tube portion are axially provided with a second through hole for the multiple first wires to pass through, the locking member holds the annular portion against the third front end of the contact assembly and the locking member, the tube portion is sleeved on the outer side of the multiple first wires, and at least a portion of the tube portion is located between the inner wall of the locking member and the multiple first wires to achieve sealing between the locking member and the multiple first wires.

In a possible implementation, a cable tie is provided inside the external tube and near the third front end of the contact assembly, the multiple first wires are fixedly arranged inside the external tube through the cable tie, the outer side of the multiple first wires is coated with a protection layer, and the multiple first wires and the protection layer pass through the cable tie and pass through the first through hole of the locking member.

In a possible implementation, a wire grouping portion is disposed at the third front end of the contact assembly, the wire grouping portion is used to group the multiple first wires into at least two groups, and the first wires in each of the at least two groups are located on the same side surface of the contact assembly.

In a possible implementation, the transmission module further includes a coating layer and a positioning assembly, where the coating layer covers the external tube, the locking member, and part of the outer side surface of the multiple first wires near the first front end, at least part of the positioning assembly is disposed in an accommodation cavity of the coating layer and near the first rear end of the transmission module, and the positioning assembly is used for fixedly connecting the extension member in the accommodation hole.

In some possible implementations, the positioning assembly includes a sealing and waterproof member, a sleeve tube, a positioning screw, and a top water seal, the sealing and waterproof member and the sleeve tube are disposed in the accommodation cavity of the coating layer and are respectively provided with a third through hole and a fourth through hole, the third through hole and the fourth through hole are axially coincide with the accommodation hole and allow the extension member to pass through, the sealing and waterproof member seals the second rear end of the sleeve tube, the sidewall of the sleeve tube is provided with a penetrating hole that penetrates from the inside to the outside, the positioning screw passes through the penetrating hole of the sleeve tube and is used for abutting against the extension member to fix the extension member, and the top water seal is disposed on the coating layer and is used for sealing the positioning screw.

The fourth front end of the coating layer is tapered to fix and seal the multiple first wires extending from the first front end of the transmission module.

The present application further provides an electrical stimulation device. The electrical stimulation device includes a pulse generator, a second wire, an extension member, an electrode, and any transmission module described above.

The pulse generator is connected to the transmission module via a first wire, and the electrode is connected to the transmission module via the second wire and the extension member connected to the second wire; or the pulse generator is connected to the transmission module via the second wire and the extension member connected to the second wire, and the electrode is connected to the transmission module via the first wire.

The transmission module and the electrical stimulation device in the present application have at least the advantages described below.

In the present application, the integral bracket is provided, and multiple partition-grooves are provided on the bracket and used for fixing the connecting members. In this manner, in the integral bracket, the assembly dimension between modules does not increase with the number of modules, and the tolerance of multiple modules during assembly can be prevented from increasing. Moreover, the provided locking member can effectively confine and hold the multiple first wires tightly, thereby preventing the following: the first wires are bent or pulled, causing the solder points to come off and fail.

### BRIEF DESCRIPTION OF DRAWINGS

FIG. 1 is a schematic view illustrating the overall structure of a transmission module according to an embodiment of the present application.
FIG. 2 is a schematic view illustrating the structure of a transmission module without a coating layer according to an embodiment of the present application.
FIG. 3 is a schematic view illustrating the structure of a transmission module without an external tube according to an embodiment of the present application.
FIG. 4 is an axial sectional view of a transmission module according to an embodiment of the present application.
FIG. 5 is a radial sectional view of a transmission module according to an embodiment of the present application.
FIG. 6 is a view illustrating the structure of a conductive member according to an embodiment of the present application.
FIG. 7 is a view illustrating the structure of an extension member according to an embodiment of the present application.
FIG. 8 is a schematic diagram illustrating the connection structure of an electrical stimulation device according to an embodiment of the present application.
FIG. 9 is a schematic diagram illustrating the connection structure of an electrical stimulation device according to another embodiment of the present application.

### Reference list

- 100: transmission module
- 101: first front end
- 102: first rear end
- 1: contact assembly
- 11: integral bracket
- 111: partition-groove
- 112: accommodation hole
- 113: conductive member
- 1131: groove
- 1132: hole
- 1133: second transition surface
- 1134: edge
- 114: wire tube
- 115: first transition surface
- 12: connecting member
- 13: branching portion
- 14: third front end
- 15: side surface
- 2: first wire
- 3: external tube
- 31: sealing sleeve
- 32: annular portion
- 33: tube portion
- 34: second front end
- 35: inner circumferential surface
- 36: second through hole
- 37: outer side surface
- 4: locking member
- 41: first through hole
- 42: inner wall
- 5: cable tie
- 6: protection layer
- 7: coating layer
- 71: accommodation cavity
- 72: fourth front end
- 8: positioning assembly
- 81: sealing and waterproof member
- 811: third through hole
- 82: sleeve tube
- 821: fourth through hole
- 822: second rear end
- 823: sidewall
- 824: penetrating hole
- 83: positioning screw
- 84: top water seal
- 9: extension member
- 10: second wire
- 200: pulse generator
- 300: electrode

### DETAILED DESCRIPTION

Example embodiments are described more fully with reference to the drawings. However, the example embodiments may be implemented in many different forms and should not be construed as limited to the embodiments set forth herein. Rather, these embodiments are provided so that the present application is thorough and complete and fully conveys the concept of the example embodiments to those skilled in the art. The same reference numerals in the drawings denote the same or similar structures, and thus the description of the same reference numerals is not repeated.

Expressions of positions and directions in the present application are described by using the drawings as examples. However, changes may be made as required, and the changes fall within the scope of the present application.

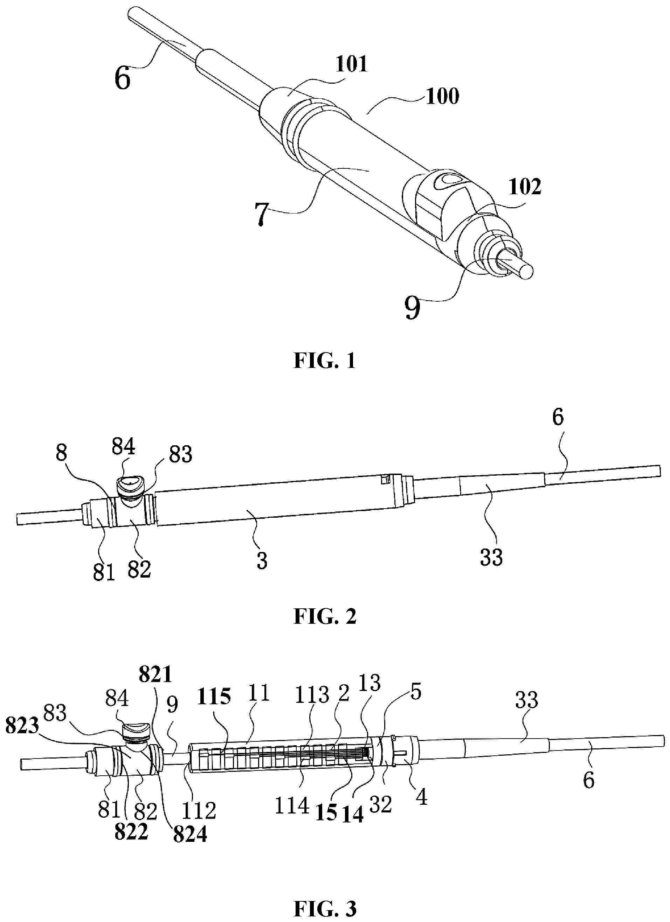

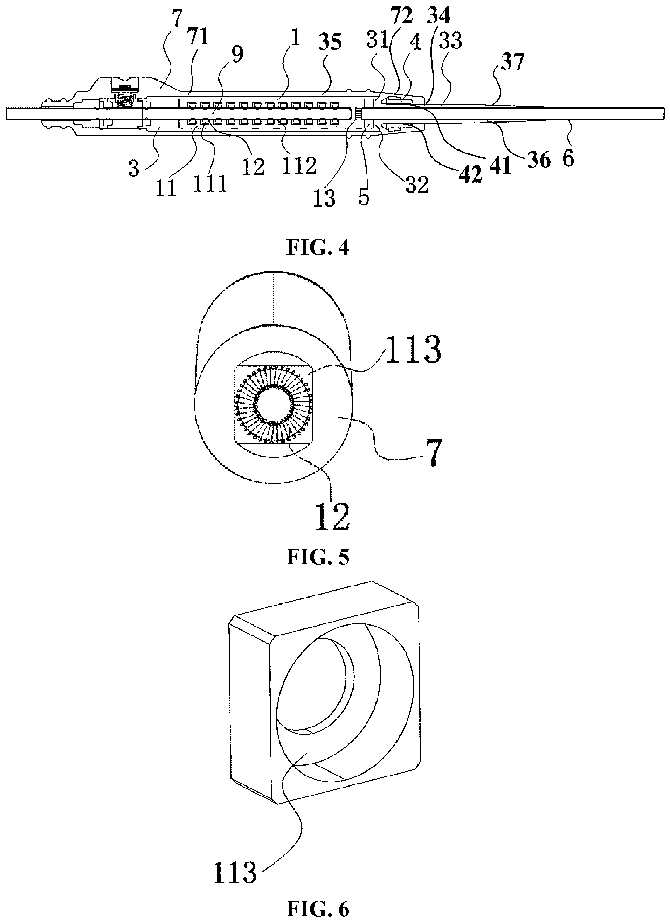

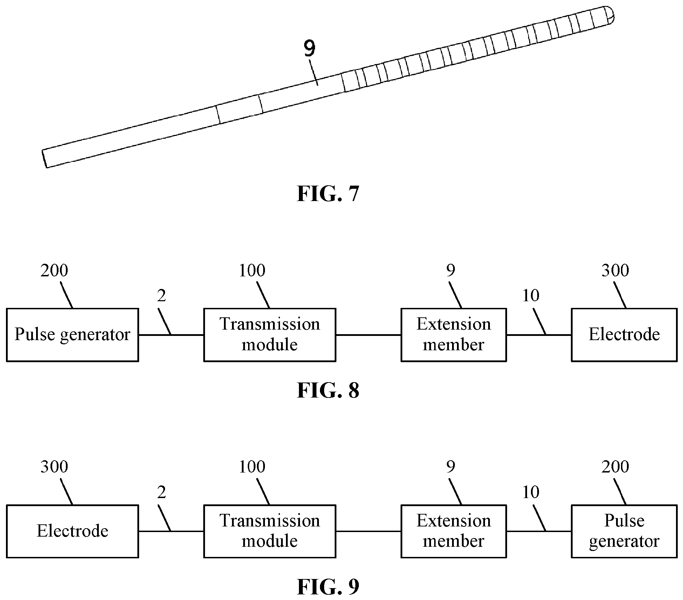

Referring to FIGS. 1 to 9, FIG. 1 is a schematic view illustrating the overall structure of a transmission module 100 according to an embodiment of the present application, FIG. 2 is a schematic view illustrating the structure of a transmission module 100 without a coating layer 7 according to an embodiment of the present application, FIG. 3 is a schematic view illustrating the structure of a transmission module 100 without an external tube 3 according to an embodiment of the present application, FIG. 4 is an axial sectional view of a transmission module 100 according to an embodiment of the present application, FIG. 5 is a radial sectional view of a transmission module 100 according to an embodiment of the present application, FIG. 6 is a view illustrating the structure of a conductive member 113 according to an embodiment of the present application, FIG. 7 is a view illustrating the structure of an extension member according to an embodiment of the present application, FIG. 8 is a schematic diagram illustrating the connection structure of an electrical stimulation device according to an embodiment of the present application, and FIG. 9 is a schematic diagram illustrating the connection structure of an electrical stimulation device according to another embodiment of the present application. The present application discloses a transmission module 100 with an integral bracket 11. The transmission module 100 has a front end 101 and a rear end 102 opposite to each other and includes a contact assembly 1, multiple first wires 2, an external tube 3, and a locking member 4.

The contact assembly 1 includes the integral bracket 11 and multiple connecting members 12. The integral bracket 11 is provided with multiple spaced-apart partition-grooves 111. The connecting members 12 are each disposed in one of the partition-grooves 111 of the integral bracket 11. The structure between two adjacent partition-grooves 111 insulates two adjacent connecting members 12 from each other. The integral bracket 11 has an accommodation hole 112, and an extension member 9 is inserted from the first rear end 102 of the transmission module 100 into the accommodation hole 112. Each first wire 2 is electrically connected to one or more connecting members 12. The external tube 3 is sleeved outside the contact assembly 1. The locking member 4 is sealingly connected to a second front end 34 of the external tube 3. The locking member 4 has a first through hole 41. The multiple first wires 2 pass through the first through hole 41 of the locking member 4 and are drawn out from the first front end 101 of the transmission module 100. The locking member 4 tightly holds the multiple first wires 2. The specific positions of the multiple connecting members 12 are determined based on the positions of the multiple partition-grooves 111 provided on the integral bracket 11. Compared with the traditional design of multiple mating modules, the mating tolerance can be effectively reduced, the multiple connecting members 12 can be more compactly arranged, a good position correspondence between the connecting members 12 and the extension member 9 can be ensured, and a good signal transmission effect can be achieved. Moreover, the locking member 4 connected to the first front end 101 of the transmission module 100 can effectively and tightly hold the multiple first wires 2 to prevent the solder points of the first wires 2 from coming off due to bending or pulling, and the locking member 4 abuts tightly against the integral bracket 11 inside the external tube 3 to prevent the integral bracket 11 from wobbling within the tube during use.

In some possible implementations, the inner side of the second front end 34 of the external tube 3 is threadedly connected to or snap-fit with the locking member 4. Internal threads are provided on the inner side of the second front end 34 of the external tube 3. For example, the locking member 4 is a screw. The locking member 4 is fixed to the second front end 34 of the external tube 3 through a threaded connection. Alternatively, a snap groove is provided on the inner side of the external tube 3, a protrusion is provided on the side surface of the locking member 4, and the locking member 4 is fixed to the inner side of the second front end 34 of the external tube 3 in a snap-fit manner. The locking member 4 clamped at the second front end 34 of the external tube 3 can abut tightly against the inner side of the external tube 3 to prevent the external tube 3 from wobbling during use, and the locking member 4 tightly holds the first wires 2 to prevent the following: the solder points of the first wires 2 come off when bent and pulled, causing damage to the transmission module 100.

In some possible implementations, the integral bracket 11 has the conductive member 113 connected in the partition-groove 111, a groove 1131 for fixing the connecting member 12 and a hole 1132 for the extension member 9 to pass through are provided on the inner side of the conductive member 113, the conductive member 113 connects the first wire 2 to the connecting member 12, a first transition surface 115 for arranging the first wires 2 is disposed on the outer surface of the integral bracket 11, part of a surface of the conductive member 113 is exposed from the first transition surface 115 of the integral bracket 11 and is used for connecting the first wire 2, and the connecting member 12 is a coil spring. One conductive member 113 is provided at the opening of each corresponding partition-groove 111 of the integral bracket 11, the inner side of the conductive member 113 is connected to the connecting member 12, and the outer side of the conductive member 113 is connected to one first wire 2. The connecting member 12 is a coil spring. The integral bracket 11 is made of insulating material. The conductive member 113 is connected in the partition-groove 111. During connection, through the elastic force of the integral bracket 11, the integral bracket 11 elastically presses and fixes the conductive member 113 within the partition-groove 111, and the first outer side surface of the conductive member 113 and the surface of the integral bracket 11 are kept being smoothly connected. For example, the conductive member 113 is made of existing metal, such as iron, copper, or aluminum. In some possible implementations, the conductive member 113 includes a wire tube 114. The wire tube 114 is connected to the conductive member 113 and correspondingly disposed on the outer side of the contact assembly 1. The wire tube 114 is used for connecting the first wire 2. The wire tube 114 is disposed on a second transition surface 1133 of the conductive member 113, and an edge 1134 of the second transition surface 1133 of the conductive member 113 smoothly transitions to the outer surface of the integral bracket 11 so that the inner circumferential surface 35 of the external tube 3 applies a force to the edge 1134 of the second transition surface 1133 of the conductive member 113 and presses the conductive member 113 into the integral bracket 11. During assembly, the conductive member 113 with a coil spring fixed inside is inserted along the partition-groove 111 of the integral bracket 11, the external tube 3 fixes the conductive member 113 in the corresponding partition-groove 111 by the pressing force from the edge 1134 of the second transition surface 1133 of the conductive member 113 acted on the conductive member 113, and after installation, the integral bracket 11 and the second transition surface 1133 are smoothly connected.

The integral bracket 11 has one or more side surfaces 15 on which the conductive members 113 are disposed. The conductive members 113 on the same side surface 15 of the integral bracket 11 are spaced apart and insulated from each other. During connection, for example, the metal end of the first wire 2 passes through the inner hole of the wire tube 114 and is welded and fixed. The inner hole of the wire tube 114 has a larger contact area when connected to the first wire 2 so that it can be ensured that the first wire 2 connected to the wire tube 114 transmits signals stably. Moreover, the integral bracket 11 is, for example, a columnar structure with two side surfaces 15, four side surfaces 15, or eight side surfaces 15. When the number of conductive members 113 on the transmission module 100 is larger, the integral bracket 11 is provided with multiple side surfaces 15, and the multiple first wires 2 are connected to the multiple side surfaces 15, respectively according to a fixed rule, thereby reducing the mutual interference between the multiple first wires 2 during operation, facilitating wiring, and preventing short circuits. When the integral bracket 11 is impacted or pressed by an external force, the annular structures of the conductive members 113 inside the integral bracket 11 have a supporting function, thereby preventing the integral bracket 11 from deforming.

In some possible implementations, the external tube 3 includes a sealing sleeve 31, and the sealing sleeve 31 includes an annular portion 32 and a tube portion 33. The annular portion 32 and the tube portion 33 are axially provided with a second through hole 36 for the multiple first wires 2 to pass through. The locking member 4 holds the annular portion 32 against a third front end 14 of the contact assembly 1 and the locking member 4. The tube portion 33 is sleeved on the outer side of the multiple first wires 2, and at least a portion of the tube portion 33 is located between an inner wall 42 of the locking member 4 and the multiple first wires 2 to achieve sealing between the locking member 4 and the multiple first wires 2. A sealing sleeve is made of rubber. For example, the annular portion 32 and the tube portion 33 are integrally formed rubber parts. The first wires 2 pass through the second through hole 36 of the tube portion 33. The outer side of the tube portion 33 passes through the first through hole 41 of the locking member 4. The tube portion 33 applies a confining force to the first wires 2 passing through the inner side of the tube portion 33, thereby bundling the multiple first wires 2 together. The locking member 4 abuts against one side annular surface of the annular portion 32. During use, the annular portion 32 of the sealing sleeve improves the sealing performance between the external tube 3 and the locking member 4, and the tube portion 33 bundles the multiple first wires 2 together to prevent the solder points of any of the first wires 2 from coming off due to pulling.

By providing the external tube 3, the first wires 2 can be confined, thereby preventing the first wires 2 from moving and improving reliability. In addition, the first wires 2 and the contact assembly 1 in the external tube 3 can be protected, thereby preventing liquid penetration and improving waterproof performance. Moreover, the external tube 3 has a supporting function so that the various parts in the contact assembly 1 maintain preset shapes, and forces outside the external tube 3 can be prevented from impacting the contact assembly 1.

In some possible implementations, a cable tie 5 is provided inside the external tube 3 and near the third front end 14 of the contact assembly 1, and the multiple first wires 2 are fixedly arranged inside the external tube 3 through the cable tie 5. The outer side of the multiple first wires 2 is coated with a protection layer 6, and the multiple first wires 2 and the protection layer 6 pass through the cable tie 5 and pass through the first through hole 41 of the locking member 4. For example, the cable tie 5 is made of insulating material. When the multiple first wires 2 pass through the first through hole 41 and the cable tie 5, the multiple first wires 2 do not interfere with each other. Moreover, when the number of first wires 2 is larger, the multiple first wires 2 may be arranged in the external tube 3 through the cable tie 5 according to a rule.

In some possible implementations, a wire grouping portion 13 is disposed at the third front end 14 of the integral bracket 11, the wire grouping portion 13 is used to group the multiple first wires 2 into at least two groups, and the first wires 2 in a same group are located on the same side surface 15 of the contact assembly 1. For example, the wire grouping portion 13 includes a slot hole. The case where the integral bracket 11 has two side surfaces 15 is used as an example. The multiple first wires 2 are divided into two groups, the two groups of the first wires 2 respectively extend from a respective side of two sides of the slot hole toward the rear end of the integral bracket 11. In this manner, the following can be prevented: the multiple first wires 2 are arranged in parallel on the same side of the external tube 3, resulting in a crowded arrangement between the first wires 2, the mutual interference between the multiple first wires 2 during operation, and short-circuiting of the first wires 2.

In some possible implementations, a coating layer 7 and a positioning assembly 8 are further included. The coating layer 7 covers the external tube 3, the locking member 4, and part of an outer side surface 37 of the multiple first wires 2 near the first front end 101, at least part of the positioning assembly 8 is disposed in an accommodation cavity 71 of the coating layer 7 and near the first rear end 102 of the transmission module 100, and the positioning assembly 8 is used for fixedly connecting the extension member 9 in the accommodation hole 112. For example, the coating layer 7 is made of rubber. The coating layer 7 is used as the connecting part connected to the outermost side of the external tube 3 to integrally cover the external tube 3, the locking member 4, and part of the outer side surface 37 of the multiple first wires 2 near the first front end 101, thereby preventing external water from penetrating into the external tube 3. The positioning assembly 8 is fixed at the first rear end 102 of the transmission module 100 to fix the extension member 9, thereby preventing the following: the extension member 9 connected to the first rear end 102 of the transmission module 100 is pulled out or detached from the transmission module 100 during use, affecting the normal use of the user.

In some possible implementations, the positioning assembly 8 includes a sealing and waterproof member 81, a sleeve tube 82, a positioning screw 83, and a top water seal 84. The sealing and waterproof member 81 and the sleeve tube 82 are disposed in the accommodation cavity 71 of the coating layer 7 and are respectively provided with a third through hole 811 and a fourth through hole 821 that are axially coincide with the accommodation hole 112 and allow the extension member 9 to pass through. The sealing and waterproof member 81 is connected to the rear end inside the coating layer 7. The sealing and waterproof member 81 is sealingly connected to a second rear end 822 of the sleeve tube 82 to prevent external water from flowing into the interior of the sleeve tube 82 through the rear end of the coating layer 7 and affecting normal operation. A sidewall 823 of the sleeve tube 82 is provided with a penetrating hole 824 that penetrates from the inside to the outside, and the axis of the penetrating hole 824 and the axis of the fourth through hole 821 on the sleeve tube 82 may be perpendicular. The positioning screw 83 passes through the penetrating hole 824 of the sleeve tube 82 and is used for abutting against the extension member 9 to fix the extension member 9. The top water seal 84 is disposed on the coating layer 7 and is used for sealing the positioning screw 83. For example, the top water seal 84 is sealingly filled with glue to seal the exposed top of the positioning screw 83. When the extension member 9 is connected to the contact assembly 1, the bottom end of the positioning screw 83 abuts tightly against the first sidewall of the protruding part of the extension member 9, thereby preventing the following: the extension member 9 is pulled out or detached from the contact assembly 1 during use by the user, causing the transmission module 100 to fail and affecting the treatment effect.

In some possible implementations, a fourth front end 72 of the coating layer 7 is tapered to fix and seal the multiple first wires 2 extending from the first front end 101 of the transmission module 100. The fourth front end 72 of the coating layer 7 is tapered to increase the confining force applied to the extended first wires 2, thereby preventing the following: the solder points of the first wires 2 come off due to bending, thereby affecting the user experience.

The present application further discloses an electrical stimulation device. The electrical stimulation device includes a pulse generator 200, a second wire 10, the extension member 9, an electrode 300, and any transmission module 100 described above.

Referring to FIG. 8, the pulse generator 200 is connected to the transmission module 100 via the first wire 2, and the electrode 300 is connected to the transmission module 100 via the second wire 10 and the extension member 9 connected to the second wire 10.

Alternatively, referring to FIG. 9, the pulse generator 200 is connected to the transmission module 100 via the second wire 10 and the extension member 9 connected to the second wire 10, and the electrode 300 is connected to the transmission module 100 via the first wire 2.

In some possible implementations, the electrical stimulation device may include at least two stimulation signal transmission methods. In one of the two stimulation signal transmission methods, the first front end 101 of the transmission module 100 is connected to the first wires 2, and the transmission module 100 is connected to the pulse generator 200 via the first wires 2. During installation, the extension member 9 of the second wire 10 connected to the electrode 300 needs to be inserted into the transmission module 100, and the extension member 9 needs to be fixed in the transmission module 100 through the contact assembly 1 before use. In the other of the two stimulation signal transmission methods, the first front end 101 of the transmission module 100 is connected to the first wire 2, the transmission module 100 is connected to the electrode 300 via the first wires 2, the pulse generator 200 is connected to the extension member 9 via the second wire 10, and the extension member 9 is inserted into the transmission module 100 and connected to the transmission module 100, thereby achieving the connection between the pulse generator 200 and the electrode 300. After the neural electrical stimulation device is implanted in the patient's body, the pulse generator 200 generates an electrical stimulation signal, the electrical stimulation signal passes through the first wire 2, the transmission module 100, and the second wire 10, and ultimately, the electrode 300 releases the electrical stimulation at the point to be stimulated, assisting in the patient's rehabilitation.

Although the embodiments of the present application have been shown and described above, it is to be understood that the preceding embodiments are exemplary and should not be construed as limiting the present application. Without departing from the principle and spirit of the present application, those of ordinary skill in the art may make any changes, modifications, substitutions, and variations on the preceding embodiments within the scope of the present application. All of these changes should fall within the scope of the claims of the present application.

## Claims

1. A transmission module, wherein the transmission module has a first front end and a first rear end opposite to each other, and the transmission module comprises:
a contact assembly comprising an integral bracket and a plurality of connecting members, wherein the integral bracket is provided with a plurality of spaced-apart partition-grooves, each of the plurality of connecting members is disposed in a respective one of the plurality of partition-grooves of the integral bracket, the plurality of connecting members are insulated from each other, and the integral bracket has an accommodation hole into which an extension member is inserted from the first rear end of the transmission module;
a plurality of first wires, each of which is electrically connected to one or more of the plurality of connecting members;
an external tube sleeved outside the contact assembly; and
a locking member sealingly connected to a second front end of the external tube, wherein the locking member has a first through hole, the plurality of first wires pass through the first through hole of the locking member and are drawn out from the first front end of the transmission module, and the locking member tightly holds the plurality of first wires.

2. The transmission module of claim 1, wherein an inner side of the second front end of the external tube is threadedly connected to or snap-fit with the locking member.

3. The transmission module of claim 1, wherein the integral bracket has a conductive member connected in each of the plurality of partition-grooves, a groove for fixing a connecting member of the plurality of connecting members and a hole for the extension member to pass through are provided on an inner side of the conductive member, the conductive member connects a first wire of the plurality of first wires to the connecting member, a first transition surface for arranging the plurality of first wires are disposed on an outer surface of the integral bracket, part of a surface of the conductive member is exposed from the first transition surface of the integral bracket and is used for connecting the first wire, and the connecting member is a coil spring.

4. The transmission module of claim 3, wherein the conductive member comprises a wire tube for connecting the first wire, the wire tube is disposed on a second transition surface of the conductive member, and an edge of the second transition surface of the conductive member smoothly transitions to the outer surface of the integral bracket so that an inner circumferential surface of the external tube applies a force to the edge of the second transition surface of the conductive member and presses the conductive member into the integral bracket.

5. The transmission module of claim 1, wherein the external tube comprises a sealing sleeve, the sealing sleeve comprises an annular portion and a tube portion, the annular portion and the tube portion are axially provided with a second through hole for the plurality of first wires to pass through, the locking member holds the annular portion against a third front end of the contact assembly and the locking member, the tube portion is sleeved on an outer side of the plurality of first wires, and at least a portion of the tube portion is located between an inner wall of the locking member and the plurality of first wires to achieve sealing between the locking member and the plurality of first wires.

6. The transmission module of claim 1, wherein a cable tie is provided inside the external tube and near a third front end of the contact assembly, the plurality of first wires are fixedly arranged inside the external tube through the cable tie, an outer side of the plurality of first wires is coated with a protection layer, and the plurality of first wires and the protection layer pass through the cable tie and pass through the first through hole of the locking member.

7. The transmission module of claim 6, wherein a wire grouping portion is disposed at the third front end of the contact assembly, the wire grouping portion is used to group the plurality of first wires into at least two groups, and first wires in a same group of the at least two groups are located on a same side surface of the contact assembly.

8. The transmission module of claim 1, further comprising a coating layer and a positioning assembly, wherein the coating layer covers the external tube, the locking member, and part of an outer side surface of the plurality of first wires near the first front end, at least part of the positioning assembly is disposed in an accommodation cavity of the coating layer and near the first rear end of the transmission module, and the positioning assembly is used for fixedly connecting the extension member in the accommodation hole.

9. The transmission module of claim 8, wherein the positioning assembly comprises a sealing and waterproof member, a sleeve tube, a positioning screw, and a top water seal, the sealing and waterproof member and the sleeve tube are disposed in the accommodation cavity of the coating layer are respectively provided with a third through hole and a fourth through hole, the third through hole and the fourth through hole are axially coincide with the accommodation hole and allow the extension member to pass through, the sealing and waterproof member seals a second rear end of the sleeve tube, a sidewall of the sleeve tube is provided with a penetrating hole that penetrates from an inside to an outside, the positioning screw passes through the penetrating hole of the sleeve tube and is used for abutting against the extension member to fix the extension member, and the top water seal is disposed on the coating layer and is used for sealing the positioning screw; and
a fourth front end of the coating layer is tapered to fix and seal the plurality of first wires extending from the first front end of the transmission module.

10. An electrical stimulation device, comprising a pulse generator, a second wire, an extension member, an electrode, and the transmission module of any one of claims 1 to 9;
wherein the pulse generator is connected to the transmission module via the first wire, and the electrode is connected to the transmission module via the second wire and the extension member connected to the second wire; or, the pulse generator is connected to the transmission module via the second wire and the extension member connected to the second wire, and the electrode is connected to the transmission module via the first wire.
